# EUROPEAN PATENT SPECIFICATION

(11) **EP 2 446 827 B1**
(45) Date of publication and mention of the grant of the patent: **01.05.2013**
(21) Application number: 11185412.1
(22) Date of filing: 17.10.2011
(51) Int. Cl.: A61B 8/14

(54) **Providing a body mark in an ultrasound system**
Bereitstellung einer Körpermarkierung in einem Ultraschallsystem
Fourniture d'une marque corporelle dans un système à ultrasons

(30) Priority: 02.11.2010 KR 20100108249
(43) Date of publication of application: 02.05.2012
(73) Proprietor: Samsung Medison Co., Ltd., Nam-myun Hongchun-gun Kangwon do 250-875 (KR)
(72) Inventor: Yoon, Sook Gon, 135-851 Seoul (KR)
(74) Representative: Schmid, Wolfgang

(56) References cited:
- EP-A1- 1 623 674
- EP-A1- 1 857 051
- EP-A1- 2 314 223
- EP-A2- 0 501 819

## Description

### TECHNICAL FIELD

The present disclosure generally relates to ultrasound systems, and more particularly to providing a body mark in an ultrasound system.

### BACKGROUND

An ultrasound system has become an important and popular diagnostic tool since it has a wide range of applications. Specifically, due to its non-invasive and non-destructive nature, the ultrasound system has been extensively used in the medical profession. Modern high-performance ultrasound systems and techniques are commonly used to produce two-dimensional or three-dimensional ultrasound images of internal features of a target object (e.g., human organs).

The ultrasound system transmits ultrasound signals to a living body, which includes a target object (e.g., a heart, a fetus, etc.), and receives ultrasound signals (i.e., ultrasound echo signals) from the living body to thereby form a 2D (two-dimensional) ultrasound image or a 3D (three-dimensional) ultrasound image. The 2D or 3D ultrasound image is displayed on a display unit.

The ultrasound system provides the ultrasound image and a body mark, which represents the target object as a picture or icon, to improve the convenience of a user and patient. The ultrasound system sets the body mark selected by the user on the ultrasound image. However, there is a problem since a different body mark from the target object may be provided. Also, the ultrasound system sets the body mark on a part of the ultrasound image. However, there is a problem that a position relationship between the body mark and the ultrasound image may not be provided clearly.

An ultrasound system according to the preamble of claim 1 and a method of providing a body mark according to the preamble of claim 7 are known from EP 1 623 674 A1.

### SUMMARY

Embodiments for providing a body mark corresponding to an application on an ultrasound image based on at least one body mark corresponding to at least one body mark in an ultrasound system are disclosed herein.

In one embodiment, by way of non-limiting example, an ultrasound system comprises the features stated in claim 1.

In another embodiment, there is a method of providing a body mark comprising the features stated in claim 7.

This summary is provided to introduce a selection of concepts in a simplified form that are further described below in the Detailed Description. This Summary is not intended to identify key or essential features of the claimed subject matter, nor is it intended to be used in determining the scope of the claimed subject matter.

### BRIEF DESCRIPTION OF THE DRAWINGS

FIG. 1 is a block diagram showing an illustrative embodiment of an ultrasound system.
FIG. 2 is a block diagram showing an illustrative embodiment of an ultrasound data acquisition unit.
FIG. 3 is a schematic diagram showing an example of a mapping table.
FIG. 4 is a flow chart showing a process of setting a body mark on an ultrasound image.
FIG. 5 is a schematic diagram showing an example of an ultrasound image and a body mark.

### DETAILED DESCRIPTION

A detailed description may be provided with reference to the accompanying drawings. One of ordinary skill in the art may realize that the following description is illustrative only and is not in any way limiting.

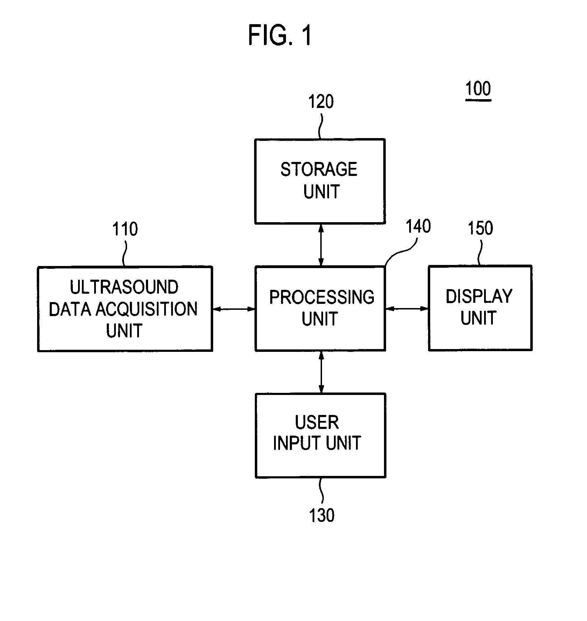

Referring to FIG 1, an ultrasound system 100 in accordance with an illustrative embodiment is shown. As depicted therein, the ultrasound system 100 includes an ultrasound data acquisition unit 110.

The ultrasound data acquisition unit 110 is configured to transmit ultrasound signals to a living body, which includes a target object. The target object includes a heart, a fetus and the like. The ultrasound data acquisition unit 110 is further configured to receive ultrasound signals (i.e., ultrasound echo signals) from the living body to acquire ultrasound data.

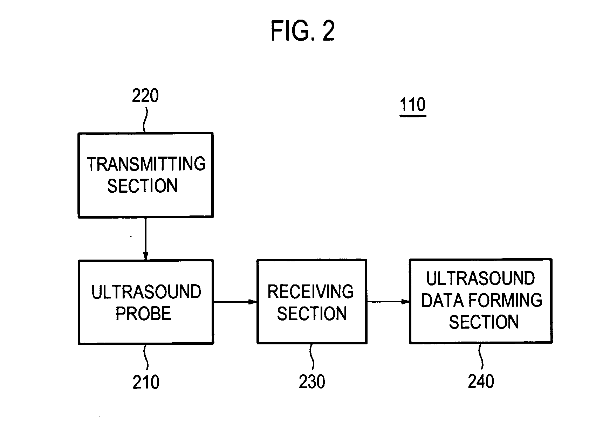

FIG 2 is a block diagram showing an illustrative embodiment of the ultrasound data acquisition unit. Referring to FIG 2, the ultrasound data acquisition unit 110 includes an ultrasound probe 210.

The ultrasound probe 210 includes a plurality of elements (not shown) for reciprocally converting between ultrasound signals and electrical signals. The ultrasound probe 210 is configured to transmit ultrasound signals to the living body. The ultrasound probe 210 is further configured to receive ultrasound echo signals from the living body to output received signals. The received signals are analog signals. The ultrasound probe 210 includes a convex probe, a linear probe and the like.

The ultrasound data acquisition unit 110 further includes a transmitting section 220. The transmitting section 220 is configured to control the transmission of the ultrasound signals. The transmitting section 220 is further configured to generate electrical signals ("transmitting signals") for obtaining an ultrasound image in consideration of the elements and focal points. Thus, the ultrasound probe 210 is configured to convert the transmitting signals into the ultrasound signals, transmit the ultrasound signals to the living body and receive the ultrasound echo signals from the living body to thereby output the received signals. The ultrasound image includes a brightness mode image. However, it should be noted herein that the ultrasound image may not be limited thereto. The transmitting section 220 includes a transmitting signal forming section (not shown), a transmitting delay time information memory (not shown), a transmitting beam former (not shown) and the like.

The ultrasound data acquisition unit 110 further includes a receiving section 230. The receiving section 230 is configured to convert the received signals provided from the ultrasound probe 210 into digital signals. The receiving section 230 is further configured to apply delays to the digital signals in consideration of the elements and the focal points to thereby output digital receive-focused signals. The receiving section 230 includes an analog-to-digital converter (not shown), a receiving delay time information memory (not shown), a receiving beam former (not shown) and the like.

The ultrasound data acquisition unit 110 further includes an ultrasound data forming section 240. The ultrasound data forming section 240 is configured to form ultrasound data corresponding to the ultrasound image based on the digital receive-focused signals. The ultrasound data includes radio frequency data. However, it should be noted herein that the ultrasound data may not be limited thereto. The ultrasound data forming section 240 is further configured to perform signal processing (e.g., gain control, etc) upon the digital receive-focused signals.

Referring back to FIG 1, the ultrasound system 100 further includes a storage unit 120. The storage unit 120 stores at least one body mark corresponding to at least one application. The application represents a diagnostic part (i.e., target object) of the living body. The body mark includes a template, which represents the diagnostic part. However, it should be noted herein that the body mark may not be limited thereto.

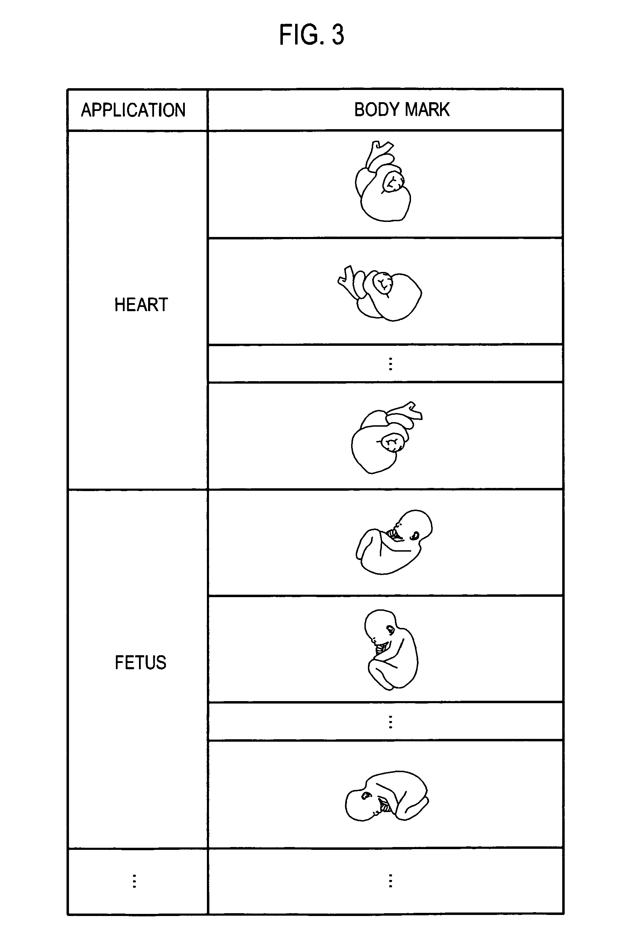

In one embodiment, the storage unit 120 stores a mapping table for providing a plurality of body marks corresponding to each of the applications (e.g., the heart, the fetus, etc.), as shown in FIG 3. The body marks have different shapes according to a position of the ultrasound probe 210, which is contacted on the surface of the living body. That is, the body marks have different shapes according to a transmitting direction of the ultrasound signals.

The ultrasound system 100 further includes a user input unit 130. The user input unit 130 is configured to receive input information from a user. In one embodiment, the input information includes first input information for selecting an application. The input information further includes second input information for adjusting the body mark. For example, the second input information includes information for adjusting a line color, a line style (e.g., a dotted line, a solid line, etc.), a line thickness, a display time (e.g., in seconds), a transparency and a position of the body mark. However, it should be noted herein that the second input information may not be limited thereto. The user input unit 130 includes a control panel, a trackball, a mouse, a keyboard and the like.

The ultrasound system 100 further includes a processing unit 140 in communication with the ultrasound data acquisition unit 110, the storage unit 120 and the user input unit 130. The processing unit 140 includes a central processing unit, a microprocessor, a graphic processing unit and the like.

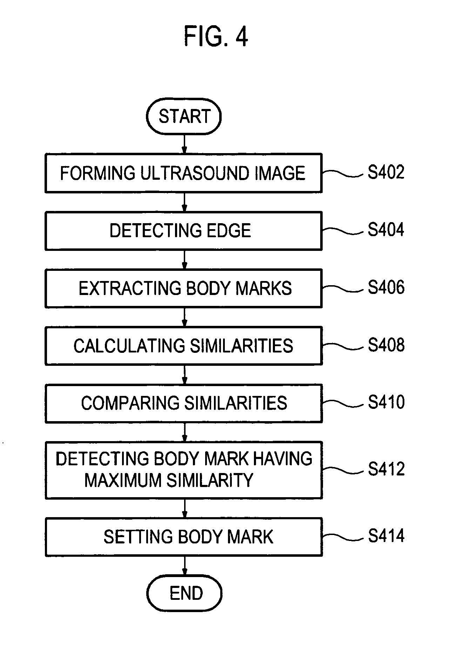

FIG 4 is a flow chart showing a process of setting the body mark on the ultrasound image. The processing unit 140 is configured to form the ultrasound image based on the ultrasound data provided from the ultrasound data acquisition unit 110, at step S402 in FIG 4.

The processing unit 140 is configured to perform edge detection upon the ultrasound image to detect an edge of the target object, at step S404 in FIG 4. The edge is detected by using an edge detecting mask such as a Sobel mask, Prewitt mask, Canny mask and the like. Also, the edge is detected by using a structure tensor.

The processing unit 140 is configured to retrieve the storage unit 120 to extract at least one body mark corresponding to the input information (i.e., first input information) provided from the user input unit 130, at step S406 in FIG 4. The body marks are extracted in the order stored in the storage unit 120.

The processing unit 140 is configured to set a body mark corresponding to an application on a predetermined position of the ultrasound image, based on the detected edge and the extracted at least one body mark.

More particularly, if at least two body marks are extracted from the storage unit 120, the processing unit 140 is configured to compare the detected edge with each of the extracted body marks to calculate similarities between the detected edge and each of the extracted body marks, at step S408 in FIG 4. The methods of calculating the similarities are well known in the art. Thus, they have not been described in detail so as not to unnecessarily obscure the present invention. The processing unit 140 is configured to the compare the calculated similarities, at step S410 in FIG 4. The processing unit 140 is configured to detect a body mark having a maximum similarity, at step S412 in FIG 4.

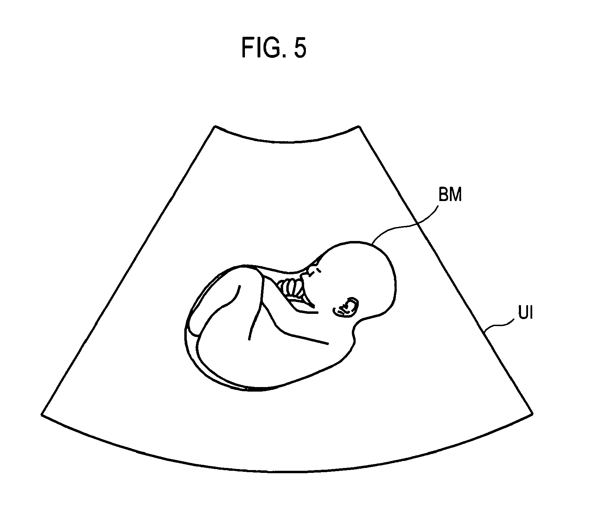

The processing unit 140 is configured to set the detected body mark on a predetermined position of the ultrasound image, at step S414 in FIG 4. In one embodiment, the processing unit 140 sets the detected body mark BM on the target object of the ultrasound image UI based on the detected edge, as shown in FIG 5.

Otherwise, if one body mark is extracted from the storage unit 120, the processing unit 140 is configured to set the extracted body mark on the target object of the ultrasound image based on the detected edge.

Optionally, the processing unit 140 is configured to adjust the body mark set on the ultrasound image, based on the input information (i.e., second input information) provided from the user input unit 130. For example, the processing unit 140 adjusts the line color, the line style, the line thickness, the display time, the transparency and the position of the body mark set on the ultrasound image, based on the second input information.

Referring back to FIG 1, the ultrasound system 100 further includes a display unit 150. The display unit 150 is configured to display the ultrasound image. The display unit 150 is further configured to display the ultrasound image and the body mark.

Although embodiments have been described with reference to a number of illustrative embodiments thereof, it should be understood that numerous other modifications and embodiments can be devised by those skilled in the art that will fall within the scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system (100), comprising:
an ultrasound data acquisition unit (110) configured to acquire ultrasound data corresponding to a living body including a target object; and
a processing unit (140) configured to form an ultrasound image based on the ultrasound data;
**characterized by**
a storage unit (120) for storing at least one body mark corresponding to at least one diagnostic part of the living body; and
the processing unit (140) being configured to perform edge detection upon the ultrasound image to detect an edge of the targetobject, detect a body mark corresponding to the edge from the storage unit (120) based on first input information for selecting a diagnostic part, and set the body mark on the ultrasound image based on the edge.

2. The ultrasound system (100) of Claim 1, wherein the at least one body mark has different shapes according to a transmitting direction of ultrasound signals.

3. The ultrasound system (100) of Claim 1, wherein the processing unit (140) is configured to:
extract at least one body mark corresponding to the first input information from the storage unit (120);
calculate at least one similarity between the edge and the extracted body mark; and
detect the body mark having a maximum similarity based on the calculated at least one similarity.

4. The ultrasound system (100) of Claim 1, further comprising:
a user input unit (130) configured to receive the first input information from a user.

5. The ultrasound system (100) of Claim 4, wherein the user input unit (130) is further configured to receive second input information for adjusting the body mark set on the ultrasound image.

6. The ultrasound system (100) of Claim 5, wherein the processing unit (140) is further configured to adjust at least one of a line color, a line style, a line thickness, a display time, a transparency and a position of the body mark set on the ultrasound image.

7. A method of providing a body mark, comprising:
a) acquiring ultrasound data corresponding to a living body including a target object;
b) forming an ultrasound image based on the ultrasound data;
**characterized by**
c) performing edge detection upon the ultrasound image to detect an edge of the target object;
d) detecting a body mark corresponding to the edge from a storage unit for storing at least one body mark corresponding to at least one diagnostic part of the living body based on first input information for selecting a diagnostic part; and
e) setting the body mark on the ultrasound image based on the edge.

8. The method of Claim 7, wherein the at least one body mark has different shapes according to a transmitting direction of ultrasound signals.

9. The method of Claim 7, wherein the step d) comprises:
extracting at least one body mark corresponding to the first input information from the storage unit;
calculating at least one similarity between the edge and the extracted body mark; and
detecting the body mark having a maximum similarity based on the calculated at least one similarity.

10. The method of Claim 7, further comprising:
f) adjusting at least one of a line color, a line style, a line thickness, a display time, a transparency and a position of the body mark set on the ultrasound image, based on second input information for adjusting the body mark set on the ultrasound image.

## Patentansprüche

1. Ultraschallsystem (100), welches Folgendes aufweist:
eine Ultraschalldatenerlangungseinheit (110), welche dafür vorgesehen ist,
einem lebenden, ein Zielobjekt beinhaltenden Körper entsprechende Ultraschalldaten zu erlangen; und
eine Verarbeitungseinheit (140), welche dafür vorgesehen ist, ein Ultraschallbild basierend auf den Ultraschalldaten zu bilden;
**gekennzeichnet durch**
eine Speichereinheit (120) zum Speichern wenigstens einer wenigstens einem zu diagnostizierenden Teil des lebenden Körpers entsprechenden Körpermarkierung; und wobei die Verarbeitungseinheit (140) dafür vorgesehen ist, eine Randerkennung an dem Ultraschallbild durchzuführen, um einen Rand des Zielobjekts zu erkennen, von der Speichereinheit (120) eine dem Rand entsprechende Körpermarkierung basierend auf ersten Eingabeinformationen zum Auswählen eines zu diagnostizierenden Teils zu erkennen und die Körpermarkierung auf dem Ultraschallbild basierend auf dem Rand festzulegen.

2. Ultraschallsystem (100) nach Anspruch 1, wobei die wenigstens eine Körpermarkierung unterschiedliche Formen entsprechend einer Übertragungsrichtung von Ultraschallsignalen aufweist.

3. Ultraschallsystem (100) nach Anspruch 1, wobei die Verarbeitungseinheit (140) für Folgendes vorgesehen ist:
Extrahieren wenigstens einer der ersten Eingabeinformation entsprechenden Körpermarkierung von der Speichereinheit (120);
Berechnen wenigstens einer Ähnlichkeit zwischen dem Rand und der extrahierten Körpermarkierung; und
Erkennen der Körpermarkierung, welche eine maximale Ähnlichkeit basierend auf der berechneten wenigstens einen Ähnlichkeit aufweist.

4. Ultraschallsystem (100) nach Anspruch 1, welches des Weiteren Folgendes aufweist:
eine Benutzereingabeeinheit (130), welche dafür vorgesehen ist, die ersten Eingabeinformationen von einem Benutzer zu empfangen.

5. Ultraschallsystem (100) nach Anspruch 4, wobei die Benutzereingabeeinheit (130) des Weiteren dafür vorgesehen ist, zweite Eingabeinformationen zum Einstellen der auf dem Ultraschallbild festgelegten Körpermarkierung zu empfangen.

6. Ultraschallsystem (100) nach Anspruch 5, wobei die Verarbeitungseinheit (140) des Weiteren dafür vorgesehen ist, wenigstens eines einer Linienfarbe, eines Linienstils, einer Liniendicke, einer Anzeigezeit, einer Transparenz und einer Position der auf dem Ultraschallbild festgelegten Körpermarkierung einzustellen.

7. Verfahren zur Erzeugung einer Körpermarkierung, welches Folgendes aufweist:
a) Erlangen von Ultraschalldaten, die einem lebenden, ein Zielobjekt beinhaltenden Körper entsprechen;
b) Bilden eines Ultraschallbilds basierend auf den Ultraschalldaten; **gekennzeichnet durch**
c) Durchführen einer Randerkennung an dem Ultraschallbild, um einen Rand des Zielobjekts zu erkennen;
d) Erkennen einer dem Rand entsprechenden Körpermarkierung von einer Speichereinrichtung zum Speichern wenigstens einer wenigstens einem zu diagnostizierenden Teil des lebenden Körpers entsprechenden Körpermarkierung basierend auf ersten Eingabeinformationen zum Auswählen eines zu diagnostizierenden Teils; und
e) Festlegen der Körpermarkierung auf dem Ultraschallbild basierend auf dem Rand.

8. Verfahren nach Anspruch 7, wobei die wenigstens eine Körpermarkierung unterschiedliche Formen entsprechend einer Übertragungsrichtung von Ultraschallsignalen aufweist.

9. Verfahren nach Anspruch 7, wobei der Schritt d) Folgendes aufweist:
Extrahieren wenigstens einer der ersten Eingabeinformation entsprechenden Körpermarkierung von der Speichereinheit;
Berechnen wenigstens einer Ähnlichkeit zwischen dem Rand und der extrahierten Körpermarkierung; und
Erkennen der Körpermarkierung, welche eine maximale Ähnlichkeit basierend auf der berechneten wenigstens einen Ähnlichkeit aufweist.

10. Verfahren nach Anspruch 7, welches des Weiteren Folgendes aufweist:
f) Einstellen wenigstens eines einer Linienfarbe, eines Linienstils, einer Liniendicke, einer Anzeigezeit, einer Transparenz und einer Position der auf dem Ultraschallbild festgelegten Körpermarkierung basierend auf zweiten Eingabeinformationen zum Einstellen der auf dem Ultraschallbild festgelegten Körpermarkierung.

## Revendications

1. Système ultrasonique (100) comportant :
une unité (110) d'acquisition de données ultrasoniques configurée pour acquérir des données ultrasoniques correspondant à un corps couché incluant un objet cible ; et
une unité de traitement (140) configurée pour former une image ultrasonique basée sur les données ultrasoniques;
**caractérisé en ce qu'**il comporte
une unité (120) de stockage configurée pour mémoriser au moins une marque corporelle correspondant à au moins une partie de diagnostic du corps couché ; et
l'unité de traitement (140) étant configurée pour effectuer la détection de bord sur l'image ultrasonique pour détecter un bord de l'objet cible, détecter une marque corporelle correspondant au bord depuis l'unité de stockage (120) basée sur des premières informations entrées pour sélectionner une partie de diagnostic, et fixer la marque corporelle sur l'image ultrasonique basée sur le bord.

2. Système ultrasonique (100) selon la revendication 1, dans lequel ladite au moins une marque corporelle a des formes différentes selon la direction de transmission des signaux ultrasoniques.

3. Système ultrasonique (100) selon la revendication 1, dans lequel l'unité de traitement (140) est configurée pour :
extraire au moins une marque corporelle correspondant aux premières informations entrées provenant de l'unité de stockage (120) ;
calculer au moins une similarité entre le bord et la marque corporelle extraite ; et
détecter la marque corporelle ayant un maximum de similarités basées sur ladite au moins une similarité calculée.

4. Système ultrasonique (100) selon la revendication 1, comprenant en outre :
une unité d'entrée de données d'utilisateur (130) configurée pour recevoir des premières informations d'entrée de l'utilisateur.

5. Système ultrasonique (100) selon la revendication 4, dans lequel l'unité d'entrée de données d'utilisateur (130) est en outre configurée pour recevoir des secondes informations d'entrée pour ajuster la marque corporelle fixée sur l'image ultrasonique.

6. Système ultrasonique (100) selon la revendication 5, dans lequel l'unité de traitement (140) est en outre configurée pour ajuster au moins une couleur de ligne, un style de ligne, une épaisseur de ligne, un temps d'affichage, une transparence et une position de la marque corporelle fixée sur l'image ultrasonique.

7. Procédé pour réaliser une marque corporelle, comprenant :
a) l'acquisition de données ultrasoniques correspondant à un corps couché incluant un objet cible ;
b) la formation d'une image ultrasonique basée sur les données ultrasoniques, **caractérisé par**
c) la réalisation d'une détection de bord sur l'image ultrasonique pour détecter un bord de l'objet cible ;
d) La détection de la marque corporelle correspondant au bord à partir de l'unité de stockage, afin de mémoriser au moins une marque corporelle correspondant à au moins une partie de diagnostic du corps allongé, basée sur les d'informations entrées pour sélectionner la partie de diagnostic ; et
e) Fixer la marque corporelle sur l'image ultrasonique basée sur le bord.

8. Procédé selon la revendication 7, dans lequel au moins une marque corporelle a différentes formes selon la direction de transmission des signaux ultrasoniques.

9. Procédé selon la revendication 7, dans lequel l'étape d) comprend :
l'extraction d'au moins une marque corporelle correspondant aux premières informations d'entrée provenant de l'unité de stockage ;
le calcul d'au moins une similarité entre le bord et la marque corporelle extraite ; et
la détection de la marque corporelle ayant un maximum de similarités basées sur ladite au moins une similarité calculée.

10. Procédé selon la revendication 7, comprenant en outre :
f) l'ajustage d'au moins une couleur de ligne, d'un style de ligne, d'une épaisseur de ligne, d'un temps d'affichage, d'une transparence et d'une position de la marque corporelle fixée sur l'image ultrasonique basés sur une seconde information d'entrée de la marque corporelle fixée sur l'image ultrasonique.
